# FASCICULE DE BREVET EUROPEEN

(11) **EP 3 277 376 B1**
(45) Date de publication et mention de la délivrance du brevet: **18.11.2020**
(21) Numéro de dépôt: 16714834.5
(22) Date de dépôt: 30.03.2016
(51) Int. Cl.: A61N 7/00, A61F 9/007, A61B 17/225

(54) **SONDE OCULAIRE DE TRAITEMENT PAR ULTRASONS**
OKULARE SONDE ZUR ULTRASCHALLBEHANDLUNG
OCULAR PROBE FOR ULTRASONIC TREATMENT

(30) Priorité: 31.03.2015 FR 1500644
(43) Date de publication de la demande: 07.02.2018
(73) Titulaire: Eye Tech Care, 69140 Rillieux-la-Pape (FR)
(72) Inventeur: CHAPUIS, Philippe, 69480 Pommiers (FR); CHARREL, Thomas, 01390 Mionnay (FR); DEVIGNE, Cedric, 01330 Villars Les Dombes (FR); LAISNEY, Nicolas, 69100 Villeurbanne (FR); RAZAVI MASHOOF, Arash, 69004 Lyon (DE)
(74) Mandataire: Be IP
(86) Numéro de dépôt international: PCT/EP2016/056898
(87) Numéro de publication internationale: WO 2016/156381

(56) Documents cités:
- WO-A1-96/28213
- WO-A2-2006/129047
- US-A- 4 484 569
- US-A1- 2011 077 514
- US-A1- 2014 276 083

## Description

### DOMAINE DE L'INVENTION

La présente invention concerne le domaine technique général des dispositifs de traitement non-invasif d'une pathologie oculaire par génération d'ultrasons focalisés ou non-focalisés, de haute ou faible intensité.

Plus particulièrement, l'invention concerne une sonde d'un dispositif de génération d'ultrasons pour le traitement d'une pathologie oculaire, comme par exemple le traitement d'un glaucome.

### ARRIERE PLAN DE L'INVENTION

Le glaucome est une neuropathie optique, c'est à dire une dégénérescence du nerf optique, ceci conduisant très souvent à une augmentation de la pression intraoculaire (PIO).

Lorsque l'humeur aqueuse n'est plus évacuée suffisamment rapidement, celle-ci s'accumule, ce qui induit une augmentation de la PIO. L'augmentation de la PIO compresse les axones dans le nerf optique et peut également compromettre la vascularisation du nerf optique. Une PIO élevée pendant une longue période peut induire une perte de vision totale.

La seule approche thérapeutique actuellement disponible pour traiter le glaucome consiste à réduire la pression intraoculaire :
- soit en améliorant le drainage d'humeur aqueuse à travers le trabéculum et le canal de Schlemm de l'œil,
- soit en réduisant la production d'humeur aqueuse par le corps ciliaire de l'œil.

On connaît du document WO 2009/103721 un dispositif pour réduire la production d'humeur aqueuse basé sur le principe de cyclo coagulation par Ultrasons Focalisés de Haute intensité qui consiste à détruire une partie des corps ciliaires afin de réduire la production d'humeur aqueuse.

Le document US 4 484 569 décrit un ensemble de transducteurs ultrasonores. L'ensemble comprend une coquille conique recouverte d'une membrane à l'une de ses extrémités, la membrane étant destinée à venir en contact avec l'œil d'un patient. L'ensemble comprend également un (ou plusieurs) transducteur(s) à l'autre extrémité de la coquille conique.

Le document WO 96/28213 décrit une sonde ultrasonore incluant un corps inséré entre un cristal ultrasonore et une sortie de la sonde remplie d'un liquide, le corps permettant de dé-focaliser le rayonnement généré par le cristal ultrasonore. La sonde comprend également une enveloppe ayant une portion conique incluant le corps. WO 2006/129047, US 2011/077514 et US 2014/276083 représentent également l'état de la technique.

Le dispositif décrit dans WO 2009/103721 permet le traitement d'un ou plusieurs secteurs de l'œil en un seul geste. Ce dispositif comprend une sonde composée d'un anneau et de moyens de génération d'ultrasons.

L'anneau présente une partie proximale destinée à être en contact avec un œil d'un patient, et une partie distale destinée à recevoir les moyens de génération d'ultrasons.

Les moyens de génération d'ultrasons présentent un profil concave. Plus précisément, les moyens de génération d'ultrasons comprennent six transducteurs en forme de segment de cylindre positionnés sur une couronne cylindrique d'axe A-A'.

Pour permettre la transmission des ultrasons vers l'œil du patient, les six transducteurs doivent être plongés dans un liquide de couplage. Le liquide de couplage est généralement conditionné dans un flacon de type goutte-à-goutte composé :
- d'un récipient contenant le liquide de couplage,
- d'un distributeur de gouttes monté sur le récipient, et
- d'un bouchon pour la fermeture du distributeur.

Une fois l'anneau positionné sur l'œil à traiter, le praticien est donc contraint de remplir l'anneau avec du liquide de couplage en appliquant une pression sur le récipient pour expulser le liquide de couplage au travers du distributeur.

Pour limiter la durée de l'étape de remplissage, le praticien applique généralement une pression très forte de sorte que le liquide est expulsé dans l'anneau sous forme de jet.

Au fur et à mesure du remplissage de l'anneau, ce jet induit des turbulences avec le liquide déjà contenu dans l'anneau, ce qui entraine la formation de bulles d'air qui peuvent venir se loger entre l'œil et les moyens de génération d'ultrasons.

L'air étant peu perméable aux ultrasons, la présence d'une bulle d'air entre l'œil et les moyens de génération d'ultrasons peut nuire à l'efficacité du traitement.

Un but de la présente invention est de proposer une solution au problème décrit ci-dessus.

### BREVE DESCRIPTION DE L'INVENTION

A cet effet, l'invention propose une sonde de traitement d'une pathologie oculaire comprenant :
o un anneau incluant un tronc de cône ayant une première extrémité adaptée pour supporter des moyens de génération d'ultrasons et une deuxième extrémité pour venir en contact avec un œil d'un patient,
o des moyens de génération d'ultrasons comportant une première base destinée à venir en regard de la première extrémité, et une deuxième base opposée à la première base,
remarquable en ce que la sonde comprend en outre un régulateur de débit pour supprimer les bulles contenues dans un fluide de couplage s'écoulant vers la première base lors du remplissage de la sonde avec ledit fluide.

Ainsi, la sonde selon l'invention comprend un régulateur de débit entre les deuxième et première bases des moyens de génération. Ce régulateur de débit comporte une conduite d'amené de fluide de couplage dont les dimensions sont adaptées pour empêcher la propagation vers la première base, des bulles contenues dans le fluide de couplage.

Avantageusement, les dimensions de la conduite d'amené de fluide sont calculées pour limiter les risques de propagation de bulles lorsque le fluide de couplage a une viscosité égale à celle de l'eau. Ceci permet de garantir que la fonction de suppression des bulles du régulateur soit effective pour tout fluide de couplage ayant une viscosité supérieure ou égale à celle de l'eau.

Comme indiqué précédemment, les bulles d'air nuisent à la propagation des ultrasons du fait de leur forte résistance acoustique.

Le régulateur de débit permet de réduire le risque de présence de ces bulles d'air entre l'œil du patient et les moyens de génération d'ultrasons, ce qui garantit toute l'efficacité de la sonde.

Des aspects de la sonde de traitement selon l'invention sont les suivants :
- l'anneau comprend une jupe coaxiale au tronc de cône, et les moyens de génération d'ultrasons comprennent une couronne, la jupe s'étendant vers l'extérieur depuis la première extrémité de sorte à envelopper la couronne ;
- le régulateur comprend une ouverture définie entre la jupe et la couronne, ladite ouverture s'étendant sur toute la hauteur de la couronne pour permettre le passage du fluide entre les deuxième et première bases ;
- l'épaisseur de l'ouverture est comprise entre 0.1 millimètre et 5 millimètres.

Des aspects préférés mais non limitatifs de la sonde de traitement selon l'invention sont les suivant :
- l'ouverture peut s'étendre sur tout le pourtour de la couronne ;
- l'épaisseur de l'ouverture peut être comprise préférentiellement entre 0.2 et 2 millimètres, et encore plus préférentiellement égale à 0.5 millimètres ;
   - la première base des moyens de génération d'ultrasons peut comprendre un traitement hydrophile ;
   - la sonde peut également comprendre un purgeur pour l'échappement des gaz lors du remplissage de la sonde avec le fluide de couplage ;
   - les moyens de génération d'ultrasons peuvent comprendre une couronne ayant un canal central, le purgeur comprenant au moins une cheminée coaxiale au canal centrale et s'étendant vers l'extérieur sur la deuxième base ;
   - l'anneau et les moyens de génération d'ultrasons peuvent être réalisés en deux pièces distinctes destinées à être assemblées, la sonde comprenant un guide pour guider le coulissement des moyens de génération d'ultrasons relativement à l'anneau ;
   - le guide peut consister en une gorge agencée sur l'anneau et un coulisseau agencé sur les moyens de génération d'ultrasons ;
   - la sonde peut de plus comprendre au moins deux buses d'aspiration sur la deuxième extrémité, les buses étant agencées de sorte à être localisées dans un plan temporal/nasal B-B' de l'œil lorsque la sonde est positionnée sur l'œil ;
   - les moyens de génération d'ultrasons peuvent comprendre un préhenseur s'étendant sur la deuxième base ;
   - l'anneau et les moyens de génération d'ultrasons peuvent être réalisés en deux pièces distinctes destinées à être assemblées, la sonde comprenant des bloqueurs pour immobiliser l'anneau relativement aux moyens de génération d'ultrasons ;
   - les bloqueurs peuvent comprendre une paire de pattes élastiques à retour de forme s'étendant à la périphérie de la deuxième base, chaque patte incluant un bourrelet pour coopérer par clippage avec un renflement ménagé sur l'anneau de sorte à immobiliser en translation l'anneau relativement aux moyens de génération d'ultrasons ;
   - chaque bourrelet peut présenter une forme en V ;
   - la sonde peut comprendre en outre au moins un butoir pour limiter le déplacement des pattes.

### BREVE DESCRIPTION DES DESSINS

D'autres avantages et caractéristiques de la sonde selon l'invention ressortiront mieux de la description qui va suivre de plusieurs variantes d'exécution, données à titre d'exemples non limitatifs, à partir des dessins annexés sur lesquels :
- la figure 1 illustre schématiquement un anneau et des moyens de génération d'ultrasons de la sonde,
- La figure 2 illustre schématiquement des yeux d'un patient,
- La figure 3 est une vue en perspective des moyens de génération d'ultrasons,
- La figure 4 illustre schématiquement la sonde une fois l'anneau et les moyens de génération d'ultrasons assemblés.

### DESCRIPTION DETAILLEE DE L'INVENTION

On va maintenant décrire différents exemples de la sonde selon l'invention en référence aux figures. Dans ces différentes figures, les éléments équivalents sont désignés par la même référence numérique.

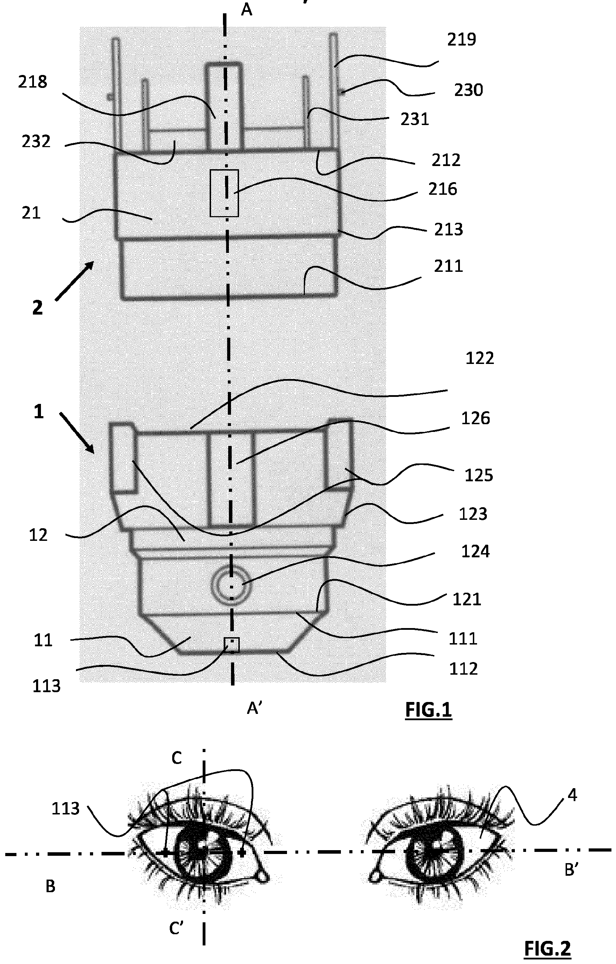

En référence aux figures 1 à 4, on a illustré un mode de réalisation de la sonde de traitement d'une pathologie oculaire.

La sonde comprend :
- un anneau 1,
- des moyens de génération d'ultrasons 2.

Les moyens de génération d'ultrasons 2 sont destinés à coopérer avec l'anneau 1. Plus précisément, l'anneau 1 constitue un logement pour les moyens de génération d'ultrasons 2.

### 1. Présentation de la Sonde 1.1. Moyens de génération d'ultrasons

Les moyens de génération d'ultrasons 2 permettent de générer une énergie ultrasonore. Par exemple, les moyens de génération d'ultrasons permettent de générer des ultrasons focalisés de haute intensité.

Ils comprennent une couronne 21 annulaire d'axe A-A' comportant :
- des première et deuxième bases opposées 211, 212 orthogonales à l'axe A-A',
- une paroi latérale externe 213 entre les première et deuxième bases, et
- une paroi latérale interne 214 définissant un canal central entre les première et deuxième bases 211, 212.

La première base 211 comporte au moins un transducteur 215 ayant un élément rayonnant pour la génération d'ultrasons.

Le profil du (ou des) élément(s) rayonnant(s) peut être adapté pour permettre l'orientation et la focalisation des ultrasons en un point donné. En variante, le transducteur 215 peut comprendre un (ou des) réflecteur(s) pour réfléchir, orienter et focaliser en un point donné les ultrasons générés par l'élément (ou les éléments) rayonnant(s).

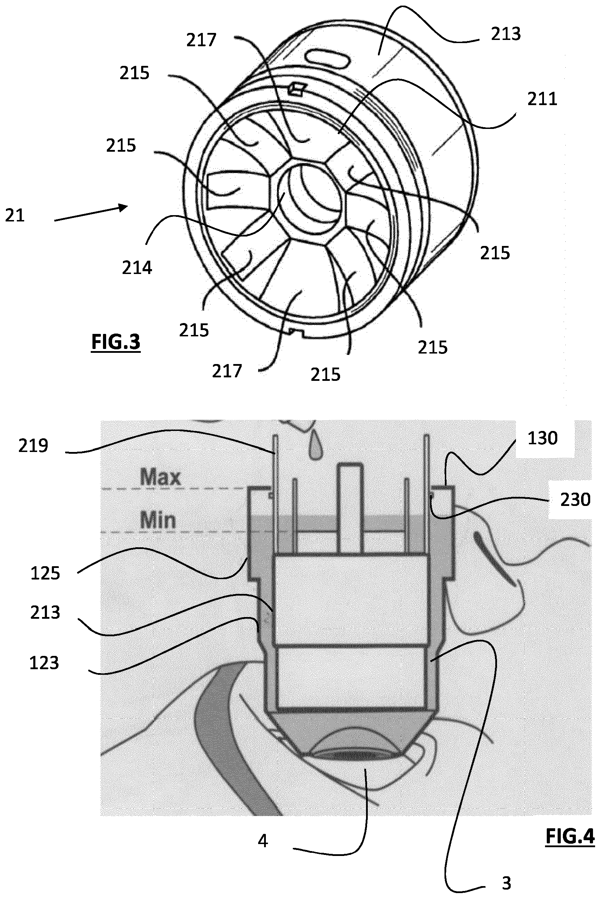

Dans le mode de réalisation illustré sur la figure 3, six transducteurs 215 s'étendent sur la première base 211 de la couronne 21.

Les transducteurs 215 sont regroupés en deux paires de trois transducteurs séparés par deux secteurs inactifs 217.

Les secteurs inactifs 217 sont localisés sur la couronne 21 de sorte à s'étendre dans un plan temporal/nasal B-B' de l'œil 4 lorsque la sonde est mise en place sur l'œil 4, ces secteurs 217 correspondant à des zones de l'œil 4 incluant la majorité des terminaisons nerveuses et vascularités.

### 1.2. Anneau

L'anneau 1 permet un positionnement adéquat et constant des moyens de génération d'ultrasons 2, aussi bien pour le centrage que pour la distance par rapport à la sclère des moyens de génération d'ultrasons 2.

L'anneau 1 comprend un tronc de cône 11 d'axe A-A', et une jupe périphérique 12 coaxiale au tronc de cône 11.

Le tronc de cône 11 est ouvert à ses deux extrémités. La grande base 111 du tronc de cône 11 - dite ***«** première extrémité »* dans la suite - comprend un berceau support destiné à recevoir les moyens de génération d'ultrasons 2. La petite base 112 du tronc de cône 11 - dite ***«** deuxième extrémité »* dans la suite - est destinée à venir en contact avec l'œil 4. La deuxième extrémité 112 peut comprendre une bride annulaire externe apte à être appliquée sur la surface externe de l'œil 4, cette bride présentant un profil concave de rayon de courbure sensiblement égal au rayon de courbure de l'œil 4.

La jupe 12 comprend une paroi annulaire 123 coaxiale au tronc de cône 11. La jupe 12 s'étend vers l'extérieur depuis la première extrémité 111 du tronc de cône 11. Plus précisément, la jupe 12 comprend des première et deuxième terminaisons ouvertes 121, 122 :
- la première terminaison 121 étant solidaire de la première extrémité 111, et
- la deuxième terminaison 122 étant opposée à la deuxième extrémité 112.

Avantageusement, la jupe 12 présente une forme complémentaire des moyens de génération d'ultrasons 2. Plus précisément, le profil intérieur de la paroi annulaire 123 est le conjugué du profil extérieur de la couronne 21.

Comme illustré à la figure 4, l'anneau 1 constitue un logement pour les moyens de génération d'ultrasons 2 :
- le tronc de cône 11 s'étend sous la couronne 21, en regard de la première base 211, et
- la jupe 12 enveloppe la couronne 21.

De préférence, la hauteur (i.e. dimension selon l'axe A-A') de la paroi annulaire 123 est supérieure à la hauteur de la couronne 21 de sorte que la deuxième terminaison 122 est localisée à une distance non nulle de la deuxième base 212.

Ceci permet de définir un espace suffisant au-dessus de la couronne 21 pour qu'elle soit totalement immergée une fois la sonde remplie avec un fluide de couplage.

### 1.3. Moyens de génération d'une dépression

La sonde peut comporter des moyens de génération d'une dépression permettant le maintien en position de l'anneau 1 sur l'œil 4 pendant toute la durée du traitement.

Les moyens de génération d'une dépression comprennent par exemple deux buses d'aspiration 113 s'étendant le long d'un diamètre de la deuxième extrémité 112.

Chaque buse 113 est reliée à un dispositif d'aspiration externe (non représenté) pour générer une dépression au niveau des buses 113 lorsque la sonde est positionnée sur l'œil 4. Ceci permet de solidariser l'anneau 1 sur l'œil 4 par effet ventouse.

Dans un mode de réalisation, chaque buse 113 consiste en une ouverture débouchant sur la deuxième extrémité 112 de sorte à venir en contact avec l'œil 4 du patient. Ceci permet de limiter les risques de perte de charge de l'aspiration (notamment par rapport à une buse de forme oblongue).

De préférence, les buses 113 sont agencées de sorte à être localisées dans le plan temporal/nasal B-B' de l'œil 4 lorsque la sonde est appliquée sur l'œil 4. Ceci permet d'améliorer l'efficacité du ventousage de l'anneau 1 sur l'œil 4.

En effet, l'œil 4 présente une forme sensiblement elliptique de plus grand rayon contenu dans le plan temporal/nasal B-B'. En agençant les buses 113 de sorte qu'elles s'étendent dans le plan temporal/nasal B-B' lorsque la sonde est mise en place, on assure un contact intime entre les buses 113 et l'œil 4, ce qui améliore l'efficacité du ventousage.

Les moyens de génération d'une dépression comprennent également :
- une conduite annulaire de circulation d'air, et
- un membre d'accès tubulaire 124
permettant le raccordement des buses 113 au dispositif d'aspiration externe par l'intermédiaire d'une tubulure de connexion.

La conduite annulaire de circulation d'air s'étend à la périphérie de la deuxième extrémité 112. Elle permet la distribution de la dépression générée aux buses 113.

Le membre d'accès 124 s'étend radialement vers l'extérieur de la paroi annulaire 123. De préférence, le membre d'accès 124 est agencé de sorte à être positionné dans le plan temporal/nasal B-B' de l'œil 4 lorsque la sonde est mise en place sur l'œil 4. Ceci permet de ne pas gêner le praticien avec la tubulure de connexion pendant le traitement.

### 1.4. Filtre

Comme indiqué précédemment, la résistance acoustique de l'air étant très importante, la présence d'une bulle d'air au niveau d'un transducteur 215 rend celui-ci inefficace. Ces bulles d'air peuvent être produites lors du remplissage de la sonde avec du fluide de couplage.

Dans le cadre de la présente invention, ce remplissage est mise en œuvre en projetant le fluide de couplage sur la deuxième base 212 de la couronne 21 après avoir assemblé l'anneau 1 avec les moyens de génération d'ultrasons 2.

Pour éviter les risques d'accumulation de bulles d'air entre les transducteurs 215 et l'œil 4, la sonde comprend un régulateur de débit entre les première et deuxième bases. Celui-ci permet d'empêcher la circulation des bulles d'air vers la première base 211.

Dans le mode de réalisation illustré à la figure 4, le régulateur de débit comprend une conduite d'amené de fluide de couplage. Cette conduite consiste par exemple en une ouverture 3 entre la paroi annulaire 123 et la paroi latérale externe 213. Les dimensions de cette ouverture 3 sont prévues de sorte à retenir les bulles sur la deuxième base 212.

L'ouverture permet également le passage du fluide de couplage versé sur la deuxième base 212 vers le tronc de cône 11. Avantageusement, au moins l'une des dimensions de l'ouverture (et notamment son épaisseur) est calculée de sorte à limiter la propagation de bulles vers la première base lorsque le fluide de couplage présente une viscosité égale à celle de l'eau.

Ainsi, on garantit que la fonction de rétention des bulles (sur la deuxième base) soit remplie par le régulateur de débit avec tout type de fluide de couplage plus visqueux que l'eau.

De préférence, la distance entre la jupe 12 et la couronne 21 est constante afin de définir une ouverture annulaire 3 entre la jupe 12 et la couronne 21.

La distance entre la jupe 12 et la couronne 21 est comprise entre 0.1 et 5 millimètres, préférentiellement entre 0.5 et 2 millimètres. Ceci permet à l'ouverture 3 de filtrer les bulles de diamètre supérieur à son épaisseur (i.e. dimension de l'ouverture selon un rayon du tronc de cône) tout en garantissant une coopération suffisante entre l'anneau 1 et les moyens de génération d'ultrasons 2.

Des cannelures peuvent être ménagées dans la paroi annulaire 123 et/ou dans la paroi latérale externe 213 pour faciliter l'écoulement du fluide de couplage vers le tronc de cône 11 lors du remplissage de la sonde. Ceci permet d'augmenter le débit d'écoulement du fluide de couplage afin d'éviter les risques de débordement lors du remplissage. Les cannelures peuvent s'étendre sur toute ou partie de la hauteur (i.e. dimension selon l'axe A-A') de la couronne 21.

Même si le régulateur de débit permet de retenir la majorité des bulles contenues dans le fluide de couplage lors du remplissage, des microbulles peuvent néanmoins circuler dans le flux de fluide s'écoulant entre les deuxième et première bases 212, 211.

C'est pourquoi la surface de la couronne 21 peut être traitée pour augmenter ses propriétés hydrophiles. Par exemple le (ou les) transducteur(s) peut (peuvent) être recouvert(s) d'une couche de matériau hydrophile. Ceci permet de limiter l'adhérence de bulles d'air sur le (ou les) transducteur(s) 215.

### 1.5. Purgeur

La sonde comprend également un purgeur pour l'évacuation de l'air contenu dans l'anneau 1 lors du remplissage de la sonde.

Le purgeur comporte une cheminée 218 coaxiale au canal central. Cette cheminée 218 s'étend au droit de la paroi latérale interne 214.

La cheminée 218 permet d'empêcher l'écoulement du fluide de couplage à travers le canal central lors du remplissage de la sonde. On contraint ainsi le fluide à s'écouler à travers l'ouverture 3 formant régulateur de débit.

Avantageusement, l'extrémité libre de la cheminée 218 comprend un trou pour permettre l'échappement de l'air. En effet, lors du remplissage de la sonde, le fluide de couplage prend progressivement la place de l'air contenu dans l'anneau 1. L'air tend à s'accumuler dans le canal central de la couronne 21. Le trou ménagé dans la cheminée permet alors à cet air de s'échapper vers l'extérieur de la sonde.

### 1.6. Moyens de préhension

La sonde comprend également des moyens de préhension pour faciliter sa manipulation par le praticien, et lui donner une indication sur les zones où positionner ses doigts.

De préférence les moyens de préhension comportent :
- un premier préhenseur disposé sur la jupe 12 pour permettre au praticien de tenir l'anneau 1 entre son pouce et son index, et
- un deuxième préhenseur disposé sur la couronne 21 pour autoriser le praticien à manipuler les moyens de génération d'ultrasons 2 entre son pouce et son index.

Le premier préhenseur consiste par exemple en deux pions 125 s'étendant radialement vers l'extérieur de la paroi annulaire 123. Avantageusement, les pions 125 peuvent être agencés sur la paroi annulaire 123 de sorte à être positionnés dans un plan supérieur/inférieur C-C' (orthogonal au plan temporal/nasal B-B') lorsque la sonde est mise en place sur l'œil 4. Ceci permet au praticien d'être dans une position ergonomique et être toujours placé à la tête du patient.

Pour améliorer la préhension de l'anneau 1, chaque pion 126 peut présenter une surface concave éventuellement striée. Ceci permet de réduire le risque de glissement accidentel entre le premier préhenseur et le gant chirurgical du praticien (qui peut dans certains cas être humide).

Le deuxième préhenseur peut consister en des extrémités libres de deux pattes 219 planes élastiques à retour de forme. Comme illustré à la figure 4, ces deux pattes 219 sont disposées en regard l'une de l'autre le long d'un diamètre de la couronne 21, et s'étendent en saillie à la périphérie de la deuxième base 212, dans le prolongement de la paroi latérale externe 213.

Une empreinte creusée préformée peut être ménagée à l'extrémité de chaque patte 219 afin de limiter les risques de glissement du gant chirurgical du praticien sur le deuxième préhenseur.

### 1.7. Guide en translation

La sonde comprend également un guide en translation pour faciliter l'assemblage des moyens de génération d'ultrasons avec l'anneau 1.

Ceci permet de garantir un positionnement précis et répétable des moyens de génération d'ultrasons 2 par rapport à l'anneau 11.

Dans le mode de réalisation illustré à la figure 1, le guide en translation consiste en :
- une gorge 126 agencée sur l'anneau 1 et
- un coulisseau 216 agencé sur les moyens de génération d'ultrasons 2.

La gorge 126 est ménagée sur la face intérieure de la paroi annulaire 123. Cette gorge 126 est adaptée pour recevoir le coulisseau 216.

Le coulisseau 216 s'étend radialement vers l'extérieur de la paroi latérale externe 213. Le coulisseau 216 peut consister en un doigt s'étendant sur une partie ou sur la totalité de la hauteur de la couronne 21.

Le guide en translation permet d'empêcher la rotation relative des moyens de génération d'ultrasons 2 par rapport à l'anneau 1 autour de l'axe A-A'.

L'utilisation d'un coulisseau 216 destiné à coopérer avec une gorge 126 permet de donner au praticien une indication sur l'orientation des moyens de génération d'ultrasons 2 par rapport à l'anneau 1, les moyens de génération d'ultrasons 2 ne pouvant pénétrer dans l'anneau 1 que selon une unique orientation.

### 1.8. Bloqueur en translation

La sonde peut également comprendre un bloqueur pour fixer de manière réversible les moyens de génération d'ultrasons 2 sur l'anneau 1.

Le bloqueur permet d'immobiliser (en translation) les moyens de génération d'ultrasons 2 une fois la sonde assemblée. Il comprend :
- deux bourrelets 230 sur les moyens de génération d'ultrasons 2,
- deux renflements 130 sur l'anneau 1.

Chaque bourrelet 230 est monté sur une patte élastique 219 respective et s'étend en saillie radiale vers l'extérieur sur toute la largeur de la patte 219.

Chaque bourrelet 230 est destiné à coopérer avec un renflement 130 respectif ménagé sur la deuxième terminaison 122 et s'étendant radialement vers l'intérieur (i.e. en direction de l'axe A-A').

### 1.9. Plaqueur

Pour limiter les jeux mécaniques entre l'anneau 1 et les moyens de génération d'ultrasons 2, la sonde peut également comprendre un plaqueur pour appliquer une force d'appui sur les moyens de génération d'ultrasons 2.

Avantageusement, une composante de cette force d'appui est parallèle à l'axe A-A' et orientée vers le tronc de cône 11 de sorte à plaquer la couronne 21 contre le berceau support du tronc de cône 11.

Dans le mode de réalisation illustré aux figures 1 à 4, un plaqueur est agencé sur chaque bourrelet 230. Ce plaqueur consiste en un angle formé entre une normale à la patte et la surface du bourrelet 230 destinée à venir en contact avec son renflement 130 associé.

### 1.10. Butoir

La sonde peut également comprendre une paire de butoir associés chacun à une patte respective 219. Ces butoirs ont pour fonction de limiter la course des pattes 219.

Ceci permet de réduire les risques de détérioration des pattes 219 par l'application d'une force de pincement trop importante lors de la préhension des moyens de génération d'ultrasons 2.

Dans le mode de réalisation illustré à la figure 4, chaque butoir consiste en une lame 231 rigide s'étendant parallèlement à sa patte associée 219. L'application d'une force de pincement sur les pattes 219 induit leur torsion vers l'axe A-A' jusqu'à une position limite où les pattes 219 viennent en contact avec les lames 231. Au-delà de la position limite, le déplacement des pattes 219 vers l'axe A-A' est empêché par les lames 231.

Pour augmenter la rigidité des lames 231, chaque butoir peut comprendre un contrefort 232.

### 2. Utilisation de la Sonde

On va maintenant décrire un exemple de procédure d'utilisation de la sonde pour traiter l'œil d'un patient allongé sur une table opératoire.

Dans une étape de la procédure, l'anneau 1 et les moyens de génération d'ultrasons 2 sont connectés à un système de commande. Plus précisément :
- l'anneau 1 est raccordé à un dispositif d'aspiration du système de commande grâce à une tubulure de connexion,
- les moyens de génération d'ultrasons 2 sont raccordés à un générateur du système de commande grâce à un câble électrique.

Dans une autre étape, le praticien agrippe l'anneau 1 en saisissant les pions 125 entre son pouce et son index. Il se positionne au niveau du front du patient et pose l'anneau 1 sur l'œil 4 en mettant en contact la deuxième extrémité 112 avec l'œil 4.

Le dispositif d'aspiration est activé pour générer une dépression au niveau des buses 113 de sorte à solidariser l'anneau 1 sur l'œil 4 par effet ventouse.

Si besoin, et avant d'activer la dépression, le praticien déplace l'anneau 1 pour le centrer sur l'œil 4.

Une fois l'anneau 1 centré sur l'œil 4, le praticien prend les moyens de génération d'ultrasons 2 en pinçant les extrémités des pattes élastiques 219 entre son pouce et son index. Il positionne la couronne 21 au-dessus de la jupe 12.

Le praticien aligne ensuite le coulisseau 216 avec la gorge 126 en faisant tourner la couronne 21 autour de l'axe A-A'. Une fois le coulisseau 216 et la gorge 126 alignés, le praticien fait coulisser la couronne 21 à l'intérieur de l'anneau 1 de sorte que la paroi annulaire 123 enveloppe la paroi latérale externe 213.

Durant le déplacement relatif de la couronne 21 par rapport à l'anneau 1, les bourrelets 230 viennent en contact avec les renflements 130. Les pattes élastiques 219 se déforment jusqu'à ce que les bourrelets 230 aient franchi le renflement 130, puis reprennent leurs positions de repos.

La couronne 21 est alors bloquée dans l'anneau 1 par clipsage grâce aux bourrelets 230 et renflements 130. La forme en V des bourrelets 230 induit l'application constante d'une force contre les renflements 130 tendant à plaquer la couronne 21 contre le berceau du tronc de cône 11 afin d'assurer un positionnement précis des moyens de génération d'ultrasons 2.

Une fois la sonde assemblée, celle-ci est remplie avec le liquide de couplage. Le praticien positionne le flacon goutte-à-goutte au-dessus de la sonde et verse le liquide sur la deuxième base 212.

Le liquide s'épand sur la deuxième base 212 et s'écoule au travers de l'ouverture 3 pour remplir l'anneau 1. La cheminée 218 empêche le liquide de pénétrer dans le canal central. Les bulles d'air de diamètre supérieur à la largeur de l'ouverture 3 (i.e. dimension de l'ouverture selon un rayon de la couronne) sont retenues au niveau de la deuxième base 212.

Au fur et à mesure du remplissage de l'anneau 1, le liquide prend progressivement la place de l'air qui s'échappe au travers du trou de la cheminée 218 via le canal central s'étendant au centre de la première base concave 211.

Lorsque la couronne 21 est totalement immergée, le praticien interrompt le remplissage et active le dispositif de contrôle pour mettre en œuvre le traitement.

En limitant les risques de présence d'une bulle d'air au niveau d'un transducteur, la sonde décrite précédemment garantit au praticien une bonne efficacité de traitement.

Le lecteur aura compris que de nombreuses modifications peuvent être apportées à l'invention décrite précédemment sans sortir matériellement des nouveaux enseignements et des avantages décrits ici. Par conséquent, toutes les modifications de ce type sont destinées à être incorporées à l'intérieur de la portée des revendications jointes.

## Revendications

1. Sonde de traitement d'une pathologie oculaire comprenant :
∘ un anneau (1) incluant un tronc de cône (11) ayant une première extrémité (111) adaptée pour supporter des moyens de génération d'ultrasons (2) et une deuxième extrémité (112) pour venir en contact avec un œil (4) d'un patient, l'anneau incluant en outre une jupe (12) coaxiale au tronc de cône (11)
∘ des moyens de génération d'ultrasons (2) incluant une couronne (21) comportant une première base (211) destinée à venir en regard de la première extrémité (111), et une deuxième base (212) opposée à la première base (211), la jupe (12) s'étendant vers l'extérieur depuis la première extrémité (111) de sorte à envelopper la couronne (21),
**caractérisée en ce que** la sonde comprend en outre un régulateur de débit (3) incluant une ouverture (3) définie entre la jupe (12) et la couronne (21), l'ouverture (3) ayant une épaisseur comprise entre 0.1 millimètre et 5 millimètres et s'étendant sur toute la hauteur de la couronne (21) pour :
∘ permettre le passage entre les deuxième et première bases (212, 211), d'un fluide de couplage ayant une viscosité supérieure ou égale à celle de l'eau favorisant la transmission des ultrasons vers l'œil du patient, et
∘ empêcher la propagation de bulles contenues dans le fluide de couplage s'écoulant vers la première base (211) lors du remplissage de la sonde avec ledit fluide.

2. Sonde selon la revendication 1, dans laquelle l'ouverture s'étend sur tout le pourtour de la couronne (21).

3. Sonde selon l'une quelconque des revendications 1 ou 2 dans laquelle l'épaisseur de l'ouverture est comprise entre 0.2 et 2 millimètres, préférentiellement égale à 0.5 millimètres.

4. Sonde selon l'une quelconque des revendications 1 à 3, dans laquelle la première base des moyens de génération d'ultrasons comprend un traitement hydrophile.

5. Sonde selon l'une quelconque des revendications 1 à 4, laquelle comprend en outre un purgeur pour l'échappement des gaz lors du remplissage de la sonde avec le fluide de couplage.

6. Sonde selon la revendication 5, dans laquelle les moyens de génération d'ultrasons (2) comprennent la couronne (21) ayant un canal central, le purgeur comprenant au moins une cheminée (218) coaxiale au canal centrale et s'étendant vers l'extérieur sur la deuxième base (212).

7. Sonde selon l'une quelconque des revendications 1 à 6, dans laquelle l'anneau (1) et les moyens de génération d'ultrasons (2) sont réalisés en deux pièces distinctes destinées à être assemblées, la sonde comprenant un guide pour guider le coulissement des moyens de génération d'ultrasons (2) relativement à l'anneau (1).

8. Sonde selon la revendication 7, dans laquelle le guide consiste en une gorge (126) agencée sur l'anneau (1) et un coulisseau (216) agencé sur les moyens de génération d'ultrasons (2).

9. Sonde selon l'une quelconque des revendications 1 à 8, laquelle comprend en outre au moins deux buses d'aspiration (113) sur la deuxième extrémité (112), les buses (113) étant agencées de sorte à être localisées dans un plan temporal/nasal (B-B') de l'œil (4) lorsque la sonde est positionnée sur l'œil (4).

10. Sonde selon l'une quelconque des revendications 1 à 9, dans laquelle les moyens de génération d'ultrasons (2) comprennent un préhenseur s'étendant sur la deuxième base (212).

11. Sonde selon l'une quelconque des revendications 1 à 10, dans laquelle l'anneau (1) et les moyens de génération d'ultrasons (2) sont réalisés en deux pièces distinctes destinées à être assemblées, la sonde comprenant des bloqueurs pour immobiliser l'anneau (1) relativement aux moyens de génération d'ultrasons (2).

12. Sonde selon la revendication 11, dans laquelle les bloqueurs comprennent une paire de pattes élastiques à retour de forme (219) s'étendant à la périphérie de la deuxième base (212), chaque patte (219) incluant un bourrelet (230) pour coopérer par clippage avec un renflement (130) ménagé sur l'anneau (1) de sorte à immobiliser en translation l'anneau (1) relativement aux moyens de génération d'ultrasons (2).

13. Sonde selon la revendication 12, dans laquelle chaque bourrelet (230) présente une forme en V.

14. Sonde selon l'une quelconque des revendications 12 ou 13, laquelle comprend en outre au moins un butoir pour limiter le déplacement des pattes (219).

## Patentansprüche

1. Sonde zur Behandlung einer Augenerkrankung, die umfasst:
∘ einen Ring (1), der einen Kegelstumpf (11) umfasst, der ein erstes Ende (111), das dazu geeignet ist, Ultraschallerzeugungsmittel (2) zu tragen, und ein zweites Ende (112), um mit einem Auge (4) eines Patienten in Kontakt zu gelangen, aufweist, wobei der Ring ferner eine Übergangsstruktur (12) umfasst, die koaxial zu dem Kegelstumpf (11) ist
∘ Ultraschall-Erzeugungsmittel (2), die einen Kranz (21) umfassen, der eine erste Basis (211), die dazu bestimmt ist, dem ersten Ende (111) gegenüberzustehen, und eine zweite Basis (212) umfasst, die der ersten Basis (211) entgegengesetzt ist, wobei die Übergangsstruktur (12) sich von dem ersten Ende (111) derart nach außen erstreckt, dass der Kranz (21) umhüllt wird,
**dadurch gekennzeichnet, dass** die Sonde ferner einen Strömungsregler (3) umfasst, der eine Öffnung (3) umfasst, die zwischen der Übergangsstruktur (12) und dem Kranz (21) definiert ist, wobei die Öffnung (3) eine Dicke zwischen 0,1 Millimeter und 5 Millimetern aufweist und sich über die gesamte Höhe des Kranzes (21) erstreckt zum:
∘ Ermöglichen des Durchgangs eines Kopplungsfluids, das eine Viskosität aufweist, die höher als oder gleich diejenige von Wasser ist, wodurch die Übertragung der Ultraschallwellen hin zum Auge des Patienten erleichtert wird, zwischen der ersten und der zweiten Basis (212, 211), und
∘ Verhindern der Ausbreitung von Bläschen, die in dem Kopplungsfluid enthalten sind, das hin zu der ersten Basis (211) strömt, beim Füllen der Sonde mit dem Fluid.

2. Sonde nach Anspruch 1, wobei die Öffnung sich über den gesamten Umkreis des Kranzes (21) erstreckt.

3. Sonde nach einem der Ansprüche 1 oder 2, wobei die Dicke der Öffnung zwischen 0,2 und 2 Millimeter beträgt und vorzugsweise gleich 0,5 Millimeter ist.

4. Sonde nach einem der Ansprüche 1 bis 3, wobei die erste Basis der Ultraschall-Erzeugungsmittel eine hydrophile Behandlung umfasst.

5. Sonde nach einem der Ansprüche 1 bis 4, die ferner einen Auslass für das Ausströmen der Gase beim Füllen der Sonde mit dem Kopplungsfluid umfasst.

6. Sonde nach Anspruch 5, wobei die UltraschallErzeugungsmittel (2) den Kranz (21) umfassen, der einen mittleren Kanal aufweist, wobei der Auslass mindestens einen Schacht (218) umfasst, der koaxial zum mittleren Kanal ist und sich auf der zweiten Basis (212) nach außen erstreckt.

7. Sonde nach einem der Ansprüche 1 bis 6, wobei der Ring (1) und die Ultraschall-Erzeugungsmittel (2) aus zwei getrennten Teilen hergestellt sind, die dazu bestimmt sind, zusammengebaut zu werden, wobei die Sonde eine Führung zum Führen des Gleitens der Ultraschall-Erzeugungsmittel (2) in Bezug auf den Ring (1) umfassen.

8. Sonde nach Anspruch 7, wobei die Führung aus einer Rille (126), die an dem Ring (1) angeordnet ist, und einem Gleitstück (216) besteht, das an den Ultraschall-Erzeugungsmitteln (2) angeordnet ist.

9. Sonde nach einem der Ansprüche 1 bis 8, die ferner an dem zweiten Ende (112) mindestens zwei Saugdüsen (113) umfasst, wobei die Düsen (113) derart angeordnet sind, dass sie sich in einer Schläfen/Nasalebene (B-B') des Auges (4) befinden, wenn die Sonde an dem Auge (4) positioniert ist.

10. Sonde nach einem der Ansprüche 1 bis 9, wobei die Ultraschall-Erzeugungsmittel (2) einen Greifer umfassen, der sich auf der zweiten Basis (212) erstreckt.

11. Sonde nach einem der Ansprüche 1 bis 10, wobei der Ring (1) und die Ultraschall-Erzeugungsmittel (2) aus zwei getrennten Teilen hergestellt sind, die dazu bestimmt sind, zusammengebaut zu werden, wobei die Sonde Sperren umfasst, um den Ring (1) in Bezug auf die Ultraschall-Erzeugungsmittel (2) festzustellen.

12. Sonde nach Anspruch 11, wobei die Sperren ein Paar von elastischen Laschen (219) mit Formgedächtnis umfassen, die sich an der Peripherie der zweiten Basis (212) erstrecken, wobei jede Lasche (219) einen Wulst (230) zum Zusammenwirken mit einer an dem Ring (1) eingerichteten Verdickung (130) durch Einrasten umfasst, derart dass der Ring (1) translatorisch in Bezug auf die Ultraschall-Erzeugungsmittel (2) festgestellt wird.

13. Sonde nach Anspruch 12, wobei jeder Wulst (230) eine V-Form aufweist.

14. Sonde nach einem der Ansprüche 12 oder 13, die ferner mindestens einen Anschlag zum Begrenzen der Verlagerung der Laschen (219) umfasst.

## Claims

1. A probe for treating an ocular pathology comprising:
∘ a ring (1) including a truncated cone (11) having a first end (111) adapted to support ultrasound generating means (2) and a second end (112) for coming into contact with an eye (4) of a patient, the ring further including a skirt (12) coaxial with the truncated cone (11)
∘ ultrasound generating means (2) including a crown (21) comprising a first base (211) intended to come in front of the first end (111), and a second base (212) opposite the first base (211), the skirt (12) extending outwardly from the first end (111) so as to envelop the crown (21),
**characterized in that** the probe further comprises a flow regulator (3) including an opening (3) defined between the skirt (12) and the crown (21), the opening (3) having a thickness comprised between 0.1 millimeter and 5 millimeters and extending over the entire height of the crown (21) for:
∘ allowing the passage between the second and first bases (212, 211) of a coupling fluid having a viscosity greater than or equal to that of water promoting the transmission of ultrasound to the patient's eye, and
∘ preventing the propagation of bubbles contained in the coupling fluid flowing to the first base (211) when filling the probe with said fluid.

2. The probe as claimed in claim 1, wherein the opening extends around the entire periphery of the crown (21).

3. The probe as claimed in any one of claims 1 or 2 wherein the thickness of the opening is comprised between 0.2 and 2 millimeters, preferentially equal to 0.5 millimeters.

4. The probe as claimed in any one of claims 1 to 3, wherein the first base of the ultrasound generating means comprises a hydrophilic treatment.

5. The probe as claimed in any one of claims 1 to 4, which further comprises a trap for the escape of gases when filling the probe with the coupling fluid.

6. The probe as claimed in claim 5, wherein the ultrasound generating means (2) comprise the crown (21) having a central channel, the trap comprising at least one chimney (218) coaxial with the central channel and extending outwardly on the second base (212).

7. The probe as claimed in any one of claims 1 to 6, wherein the ring (1) and the ultrasound generating means (2) are made in two separate parts intended to be assembled, the probe comprising a guide for guiding the sliding of the ultrasound generating means (2) relative to the ring (1).

8. The probe as claimed in claim 7, wherein the guide consists of a groove (126) arranged on the ring (1) and a slide (216) arranged on the ultrasound generating means (2).

9. The probe as claimed in any one of claims 1 to 8, which further comprises at least two suction nozzles (113) on the second end (112), the nozzles (113) being arranged so as to be located in a temporal/nasal plane (B-B') of the eye (4) when the probe is positioned on the eye (4).

10. The probe as claimed in any one of claims 1 to 9, wherein the ultrasound generating means (2) comprise a gripper extending on the second base (212).

11. The probe as claimed in any one of claims 1 to 10, wherein the ring (1) and the ultrasound generating means (2) are made in two separate parts intended to be assembled, the probe comprising clamps for immobilizing the ring (1) relative to the ultrasound generating means (2).

12. The probe as claimed in claim 11, wherein the clamps comprise a pair of shape-memory elastic tabs (219) extending at the periphery of the second base (212), each tab (219) including a bead (230) for cooperating by clipping with a bulge (130) provided on the ring (1) so as to immobilize the ring (1) in translation relative to the ultrasound generating means (2).

13. The probe as claimed in claim 12, wherein each bead (230) is V-shaped.

14. The probe as claimed in any one of claims 12 or 13, which further comprises at least one stop for limiting the displacement of the tabs (219).
